# FASCICULE DE BREVET EUROPEEN

(11) **EP 3 503 853 B1**
(45) Date de publication et mention de la délivrance du brevet: **29.07.2020**
(21) Numéro de dépôt: 17768488.3
(22) Date de dépôt: 23.08.2017
(51) Int. Cl.: A61F 5/01

(54) **ORTHÈSE DE MAINTIEN DU GENOU ADAPTÉE À UNE POSITION ASSISE PROLONGÉE**
KNIEORTHESE GEEIGNET FÜR EINE LANGE SITZENDE POSITION
KNEE ORTHOSIS ADAPTED FOR A PROLONGED SITTING POSITION

(30) Priorité: 29.08.2016 FR 1658001
(43) Date de publication de la demande: 03.07.2019
(73) Titulaire: Millet Innovation, 26270 Loriol sur Drome (FR)
(72) Inventeur: MILLET, Damien, 26000 Valence (FR)
(74) Mandataire: de Roquemaurel, Bruno
(86) Numéro de dépôt international: PCT/FR2017/052267
(87) Numéro de publication internationale: WO 2018/042108

(56) Documents cités:
- EP-A1- 0 229 577
- WO-A1-2014/184459
- DE-A1- 4 322 028
- DE-U1- 9 203 328
- FR-A1- 2 607 384
- US-A- 5 925 010
- US-B1- 6 592 539

## Description

La présente invention concerne une orthèse destinée à assurer le maintien du genou. La présente invention s'applique notamment, mais non exclusivement au maintien du genou, ne nécessitant pas une immobilisation de l'articulation. Une telle orthèse peut être utilisée pour prévenir des douleurs chroniques, ou la suite d'une entorse légère, ou encore lors d'une reprise d'activité à la suite d'un traumatisme.

Il existe des orthèses de genou ou genouillères comprenant des "pelotes rotuliennes" destinées à assurer un certain maintien de la rotule, ou des pelotes de massage pour masser certains muscles. De telles orthèses sont décrites par exemple dans les demandes de brevet US 2006/0041214, US 2010/0036303, et US 2011/0160631. Les orthèses décrites dans ces documents comprennent un élément annulaire en mousse ou en silicone, destiné à entourer la rotule pour en assurer le maintien.

Ces orthèses présentent plusieurs défauts. Elles sont relativement épaisses et lourdes notamment parce qu'elles sont réalisées à l'aide de machines à tricoter qui permettent d'obtenir uniquement des tissus tricotés qui ne peuvent pas être aussi fins que les tissus tissés, c'est-à-dire comportant un fil de chaine et un fil de trame. En raison de la masse du tissu tricoté, relativement élevée, il peut être nécessaire de prévoir des armatures latérales pour éviter qu'elles s'effondrent sur elles-mêmes. En raison de leur épaisseur relativement importante et de la présence d'armatures, elles sont inconfortables sous un pantalon, voire inadaptées au port d'un pantalon étroit. Lors de flexions répétées du genou, par exemple en situation de course, elles ont tendance à glisser le long de la cuisse et de la jambe, notamment en raison de leur masse. En situation de genou fléchi, les plis qui se forment inévitablement dans le pli poplité, à l'arrière du genou, se superposent et peuvent former une épaisseur de plusieurs millimètres, ce qui peut entraîner une gêne de l'utilisateur, voire des douleurs. Elles nécessitent souvent l'usage de bandes de serrage avec boucles et crochets pour assurer un maintien suffisant sur la cuisse et sous le genou.

Dans la demande de brevet WO 2014/184459, qui décrit l'état de la technique le plus proche, le Demandeur a proposé une orthèse comprenant un manchon élastique, conformé pour exercer des forces de contention sur la jambe de part et d'autre et sur le genou, et une plaquette en un gel polymère viscoélastique fixée sur une face interne du manchon. La plaquette comprend une partie annulaire conformée pour entourer la rotule, et une languette s'étendant depuis un bord extérieur de la partie annulaire, dans une direction axiale du manchon. La plaquette présente une adhérence avec la peau telle que, sous l'effet des forces de contention exercées par le manchon, lorsque le manchon est étiré longitudinalement, elle reste étirée et applique localement des forces de maintien en direction du centre de la rotule, et des forces de rappel dans l'axe de la jambe.

Cette orthèse s'avère efficace pour maintenir le genou durant une pratique sportive. En revanche, comme les autres orthèses précitées, elle ne peut pas être portée toute une journée, en particulier pendant de longues périodes en position assise, c'est-à-dire lorsque l'articulation du genou est maintenue fléchie à plus de 80°. En effet, dans cette position, les forces appliquées par l'orthèse à la rotule peuvent provoquer à la longue des douleurs insupportables. Par ailleurs, l'adhérence du gel polymère sous le manchon applique à la peau des forces de cisaillement qui peuvent produire à la longue des brûlures.

Il existe donc un besoin pour une orthèse de genou adaptée à être portée toute une journée d'activités ordinaires, incluant de longues périodes en position assise, sans produire d'effets indésirables.

Des modes de réalisation concernent une orthèse de genou comprenant : un manchon en tissu tissé élastique, conformé pour exercer des forces de contention sur un membre inférieur de part et d'autre et sur le genou, une plaquette comprenant une couche viscoélastique, la plaquette étant fixée sur une face interne du manchon de manière à ce que la couche viscoélastique soit en contact direct avec la peau sur le genou, la plaquette comprenant une partie annulaire conformée pour entourer la rotule du genou, et une languette distale s'étendant depuis un bord extérieur de la partie annulaire, dans une direction axiale du manchon, la couche viscoélastique présentant une adhérence avec la peau telle que, sous l'effet des forces de contention exercées par le manchon, lorsque le manchon est étiré longitudinalement, la plaquette s'étire et reste étirée en appliquant localement à la partie de membre sous-jacente des forces de maintien en direction du centre du genou, et des forces de rappel dans l'axe du membre, et des ancrages proximal et distal, pour maintenir des parties proximale et distale du manchon en des positions fixes sur le membre inférieur. Selon un mode de réalisation, le manchon est formé de pièces de tissu comprenant : une pièce antérieure sur laquelle est fixée la plaquette, une pièce postérieure opposée à la pièce antérieure, et présentant un module d'élasticité suivant la direction axiale du manchon, inférieur à celui de la pièce antérieure, et deux pièces latérales fixées chacune à un bord latéral de la pièce antérieure et à un bord latéral de la pièce postérieure, et présentant un module d'élasticité suivant la direction axiale du manchon, supérieur à celui de la pièce antérieure.

Selon un mode de réalisation, le manchon comprend un manchon proximal et un manchon distal, en tissu élastique, partiellement recouverts d'une couche adhérente à la peau, disposée sur une face interne des manchons proximal et distal, pour venir en contact direct avec la peau et assurer un ancrage sur la peau de bords proximal et distal du manchon, sous l'effet des forces de contention, le manchon proximal étant fixé à un bord proximal de chacune des pièces antérieure, postérieure et latérales, et le manchon distal étant fixé à un bord distal de chacune des pièces antérieure, postérieure et latérales,

Selon un mode de réalisation, l'orthèse présente au moins l'une des caractéristiques suivantes : le manchon proximal présente une largeur comprise entre 70 et 80 mm, et la couche adhérente formée sur le manchon proximal, présente une largeur comprise entre 50 et 60 mm, le manchon distal présente une largeur comprise entre 40 et 50 mm, et la couche adhérente formée sur le manchon distal, présente une largeur comprise entre 20 et 30 mm, les couches adhérentes formées respectivement sur les manchons proximal et distal présentent une masse surfacique comprise entre 18 et 22 µg/cm², les manchons proximal et distal sont réalisés dans un tissu élastique ne comportant pas de fil mousse.

Selon un mode de réalisation, la plaquette est fixée au manchon par un secteur angulaire proximal et un secteur angulaire distal incluant la languette, la plaquette présentant des secteurs angulaires latéraux non fixés au manchon.

Selon un mode de réalisation, la couche en gel polymère de la plaquette présente une épaisseur comprise entre 0,25 et 0,5 mm, et les secteurs angulaires proximal, latéraux et distal s'étendent chacun sur sensiblement un quart de la circonférence de la partie annulaire de la plaquette.

Selon un mode de réalisation, la couche en gel polymère de la plaquette présente une épaisseur comprise entre 0,35 et 0, 45 mm, et les secteurs angulaires latéraux présentent chacun une étendue 4 à 5 fois plus grande que le secteur angulaire proximal et 1,5 à 2 fois plus grande que le secteur angulaire distal sans la languette.

Selon un mode de réalisation, la partie annulaire de la plaquette présente entre des bords intérieur et extérieur, une largeur dans le secteur angulaire proximal, comprise entre 2,2 et 2,8 cm, et une largeur dans les secteurs angulaires latéraux, comprise entre 2,7 et 3,3 cm.

Selon un mode de réalisation, la couche en gel polymère de la plaquette est réalisée en un gel de silicone obtenu par polymérisation au moins partielle d'un mélange d'huiles de polydiméthylsiloxane.

Selon un mode de réalisation, la plaquette comprend une couche en tissu élastique fixée sur la couche en gel polymère.

Selon un mode de réalisation, la languette de la plaquette est conformée de manière à couvrir la tubérosité tibiale, l'orthèse étant adaptée pour être utilisée indifféremment sur un membre inférieur droit ou gauche.

Selon un mode de réalisation, l'orthèse présente au moins l'une des caractéristiques suivantes : la pièce antérieure du manchon présente une épaisseur comprise entre 0,4 et 0,5 mm, les pièces latérales présentent une épaisseur comprise entre 0,3 et 0,4 mm, et la pièce postérieure présente une épaisseur comprise entre 0,2 et 0,3 mm.

Selon un mode de réalisation, les pièces formant le manchon présentent des modules d'élasticité en présence d'un allongement à 40%, suivant l'axe longitudinal du manchon, compris entre 1,75 et 2 N pour la pièce antérieure, compris entre 1,7 N et 3 N pour les pièces latérales, et compris entre 1,7 et 1,8 N pour la pièce postérieure.

Selon un mode de réalisation, les pièces formant le manchon présentent des modules d'élasticité en présence d'un allongement à 40%, suivant un axe transversal du manchon, compris entre 1,75 et 2 N pour la pièce antérieure, compris entre 1,7 N et 3 N pour les pièces latérales, et compris entre 1,7 et 1,8 N pour la pièce postérieure.

Selon un mode de réalisation, les pièces antérieure et postérieure s'étendent sur environ un tiers de la circonférence du manchon et les pièces latérales s'étendent sur environ un sixième de la circonférence du manchon, à 10% près.

Des exemples de réalisation de l'invention seront décrits dans ce qui suit, à titre non limitatif en relation avec les figures jointes parmi lesquelles :
les figures 1 et 2 sont des vues avant et arrière d'une orthèse de genou, selon un mode de réalisation,
les figures 3 et 4 sont des vues avant et trois-quarts arrière de l'orthèse de la figure 1, placée sur un membre inférieur droit,
les figures 5A, 5B sont des vues en coupe schématiques de l'orthèse selon des modes de réalisation, la figure 5A étant une vue en coupe transversale selon un plan AA', et la figure 5B étant une vue en coupe longitudinale selon un plan BB', les plans AA' et BB' étant montrés sur la figure 1,
la figure 6 représente une plaquette de l'orthèse, comprenant une couche en gel polymère, selon un mode de réalisation,
les figures 7A, 7B représentent la plaquette de l'orthèse, selon un autre mode de réalisation, la plaquette étant représentée dans deux configurations, respectivement le genou en extension et le genou en flexion,
la figure 8 est une vue en coupe transversale du genou droit, revêtu de l'orthèse,
les figures 9A, 9B, 9C sont des vues en coupe sagittale des os d'un membre inférieur droit (os iliaque, fémur, rotule, tibia), respectivement genou en extension, en flexion à 90°, et en flexion à 90° avec l'orthèse superposée.

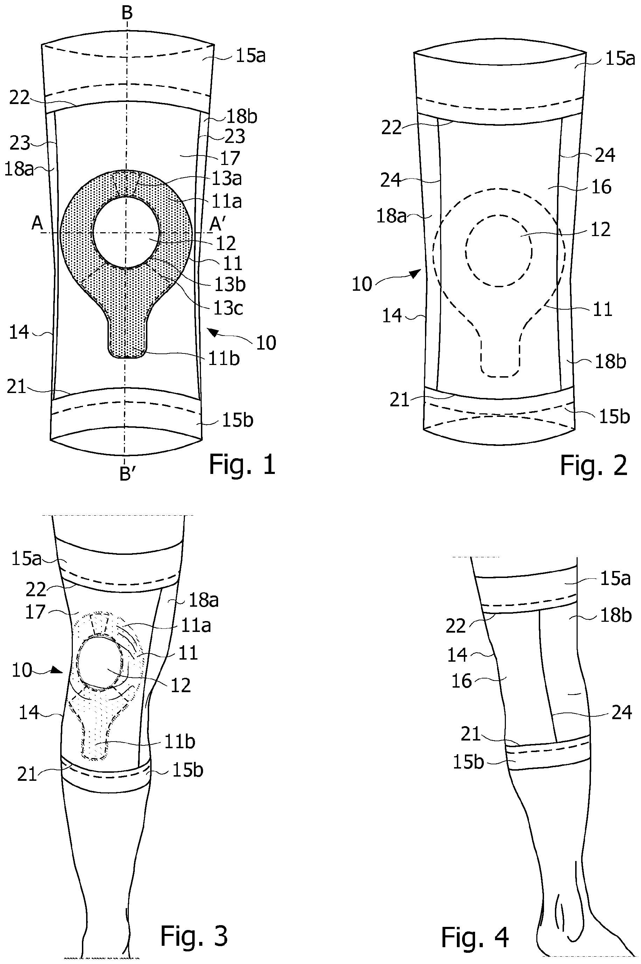

Les figures 1 à 4, 5A et 5B représentent une orthèse de genou 10 selon un mode de réalisation, les figures 3 et 4 représentant l'orthèse 10 mise en place sur un membre inférieur droit. La figure 1 représente l'orthèse dans une configuration retournée, la face visible étant celle destinée à venir en contact avec la peau. L'orthèse 10 comprend un manchon élastique 14 et une plaquette 11 comprenant une couche en un gel polymère viscoélastique, fixée sur la face du manchon 14 destinée à venir en contact avec la peau. Le manchon 14 est conformé pour exercer des forces de contention sur la cuisse, le genou et la jambe. A cet effet, le manchon 14 présente une forme tubulaire ayant un diamètre variable dans la direction longitudinale du manchon, adapté aux diamètres du bas de la cuisse, du genou et du haut de la jambe, de manière à obtenir des forces de contention souhaitées dans ces différentes parties du membre inférieur. Le manchon 14 est conformé de manière à exercer des forces de contention conformes aux normes en vigueur.

Selon un mode de réalisation, le manchon 14 est réalisé en plusieurs pièces centrales 17, 18a, 18b, 16 sensiblement de même longueur, une pièce proximale 15a en forme de manchon et une pièce distale 15b formant également un manchon. Les pièces centrales comprennent une pièce antérieure 17 sur laquelle est fixée la plaquette 11, deux pièces latérales 18a, 18b, une pièce postérieure 16,

Selon un mode de réalisation, les pièces antérieure 17 et postérieure 16 s'étendent sur environ un tiers de la circonférence du manchon 14 et les pièces latérales 18a, 18b s'étendent sur environ un sixième de la circonférence du manchon, à 10% près.

Selon un mode de réalisation, la pièce antérieure 17 (la pièce la plus sollicitée parmi les pièces 17, 18a, 18b, 16, lors de la flexion du genou) présente un module d'élasticité (module de Young) suivant l'axe du manchon 14, inférieur à celui des pièces latérales 18a, 18b, pour limiter la gêne susceptible d'apparaitre en raison du port de l'orthèse durant une longue période en position assise. Comme la pièce postérieure 16 couvre un emplacement correspondant au pli ou creux poplité du genou, elle est réalisée dans un matériau élastique présentant une faible épaisseur par exemple inférieure à 0,3 mm. Ainsi, on évite la formation de surépaisseurs résultant d'une superposition de plis, susceptibles d'induire une gêne ou une douleur lorsque le genou est fléchi en position assise ou accroupie. En effet, au-delà d'un millimètre d'épaisseur, ces surépaisseurs peuvent induire des irritations et même des brûlures. La pièce 16 peut être fixée aux autres pièces 18a, 18b, 15a, 15b formant le manchon 14 de manière à rester en tension, quelle que soit la flexion du genou, sans introduire de jeu de forces parasites.

Selon un mode de réalisation, les pièces 15a, 15b assurent le maintien du manchon 14 sur la cuisse et sur la jambe, pour éviter que le manchon 14 glisse le long de la jambe, soit vers le bas, soit vers le haut. A cet effet, les faces des pièces 15a, 15b destinées à venir en contact avec la peau sont au moins partiellement recouvertes d'une couche adhérente 19a, 19b assurant l'ancrage sur la peau des bords proximal et distal du manchon 14. Les couches adhérentes 19a, 19b présentent une adhérence avec la peau supérieure à celle du manchon 14, dépendant à la fois de leur adhérence intrinsèque, de leur surface en contact avec la peau et des forces de compression exercées par l'ensemble de la pièce 15a, 15b et de la couche adhérente 19a, 19b. Les couches adhérente 19a, 19b peuvent être continues ou discontinues. Ainsi, elles peuvent par exemple être réalisées sous la forme d'une couche, par exemple déposée par enduction, ou être réalisées sous la forme de plots. La couche et les plots peuvent être par exemple réalisés en un gel polymère tel qu'un gel de silicone, choisi pour sa propriété d'adhérence avec la peau. La largeur de chacune des couches adhérentes 19a, 19b et l'effet de contention exercé sur la cuisse ou sur la jambe par les pièces 15a, 15b peuvent être adaptés à la tenue souhaitée du manchon 14 sur la jambe tout en évitant d'exercer sur le membre des forces de contention excessives. Ainsi, Les forces d'adhésion à la peau produisant l'ancrage du haut et du bas de l'orthèse sur la peau peuvent être fixées à une valeur supérieure aux forces mises en jeu par la flexion de la jambe, compte tenu de l'élasticité de la pièce 17, afin d'éviter tout mouvement entre les couches adhérentes 19a, 19b et la peau, induisant à la longue des brûlures de contact. Les couches adhérentes 19a, 19b peuvent être réalisées dans en un gel polymère tel qu'un gel de silicone obtenu par polymérisation d'un mélange d'huiles de polydiméthylsiloxane.

Les pièces 17, 18a, 18b, 16, 15a et 15b peuvent être réalisées dans des tissus élastiques assemblés pour former le manchon 14, par exemple par des coutures 21, 22, 23, 24. La pièce antérieure 17 est fixée aux pièces latérales 18a, 18b par des coutures 23. La pièce postérieure 16 est fixée aux pièces latérales 18a, 18b par des coutures 24. La pièce proximale 15a est fixée au manchon 14 (formé par les pièces 17, 18a, 18b, 16) par une couture 22. La pièce distale 15b est fixée au manchon 14 par une couture 21. Selon un mode de réalisation, les pièces 17, 18a, 18b et 16 formant le manchon 14 sont assemblées bord à bord afin de ne pas former de surépaisseur.

La structure de genouillère qui vient d'être décrite, avec un manchon central 14 en quatre parties 17, 18a, 18b, 16, et des manchons proximal 15a et distal 15b, présente les avantages d'exercer des forces adaptées en fonction de la zone où elles s'appliquent et d'être simple à fabriquer. En particulier l'usage de pièces de tissu différentes pour réaliser le manchon 14 permet d'ajuster la raideur de chacune des pièces du manchon 14 autour de jambe et de la cuisse, en fonction de l'étirement de cette pièce durant la flexion du genou. Ainsi, la pièce 17 est amenée à être le plus étirée lorsque le genou est fléchi, tandis que la pièce 16 n'est pas sollicitée lors de ce mouvement.

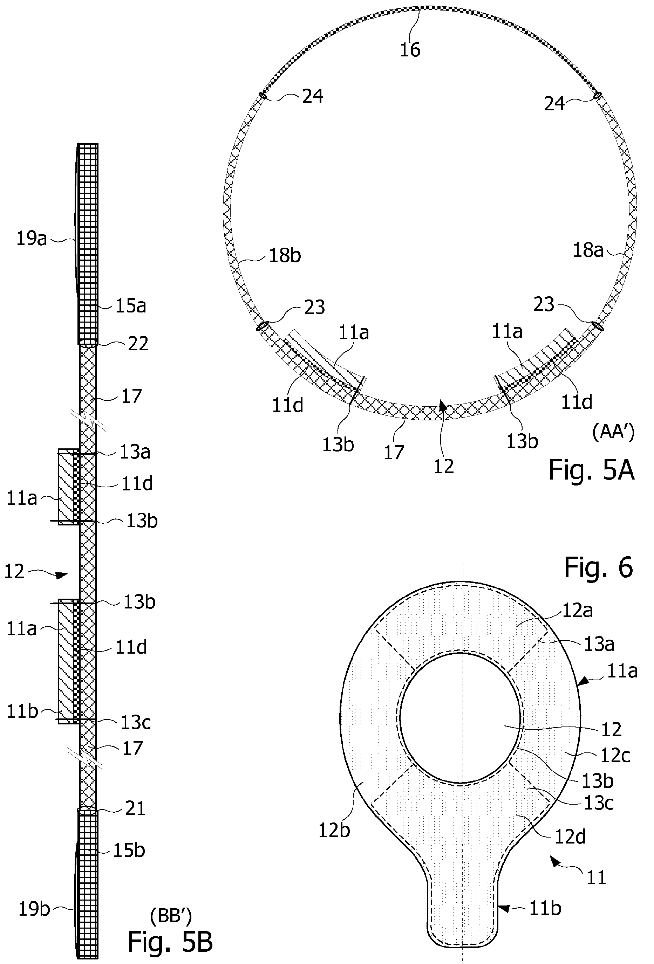

La figure 6 représente la plaquette 11, selon un mode de réalisation. La plaquette 11 comprend une partie annulaire 11a avec une ouverture centrale 12, et une languette 11b s'étendant depuis un bord extérieur de la partie annulaire. Sur les figures 1 et 3, la plaquette est fixée sur la face interne du manchon 14 en un emplacement tel que la partie annulaire 11a puisse entourer la rotule, et la languette 11b puisse couvrir la tubérosité tibiale antérieure (figure 3). La languette 11b s'étend donc dans une direction distale du manchon 14. L'ouverture 12 présente une forme circulaire ou elliptique ayant des dimensions légèrement inférieures à celles de la rotule. La partie annulaire 11a présente une largeur (entre ses bords intérieur et extérieur) comprise entre 2 et 4 cm. La languette 11b présente des dimensions légèrement supérieures à celles de la tubérosité tibiale. La languette 11b peut présenter une largeur suffisante pour éviter d'avoir à réaliser une orthèse différente pour les genoux droit et gauche, malgré le fait que la tubérosité tibiale antérieure ne soit pas centrée par rapport à un axe longitudinal de la jambe passant par le centre de la rotule.

La plaquette 11 présente une adhérence avec la peau telle que, sous l'effet des forces de contention exercées par le manchon 14, lorsque le manchon est étiré longitudinalement, elle reste étirée et applique localement à la peau des forces de traction parallèles à la surface de la peau, en direction du centre de la partie annulaire.

La plaquette 11 peut être fixée sur le manchon 14 (sur la pièce 17) par des coutures. Dans l'exemple des figures 1, 3 et 6, la plaquette 11 est fixée par des coutures 13a, 13b 13c. La couture 13a est formée le long d'une partie proximale du bord externe de la partie annulaire 11a sur un secteur angulaire proximal de la partie annulaire 11a. Les extrémités de la couture 13a peuvent rejoindre le bord interne de la partie annulaire, mais cela n'est pas indispensable. La couture 13b est formée le long de la totalité du bord interne de la partie annulaire 11a. La couture 13c est formée le long d'une partie distale du bord externe de la plaquette 11 incluant le bord de la languette 11b, sur un secteur angulaire distal de la partie annulaire 11a. Les extrémités de la couture 13c peuvent rejoindre le bord interne de la partie annulaire, mais cela n'est pas indispensable. En d'autres termes, les coutures 13a et 13c divisent la plaquette à partir de la partie annulaire 11a en quatre secteurs 12a à 12d (figure 6), à savoir un secteur proximal 12a fixé à la pièce 17, un secteur distal 12d fixé à la pièce 17, incluant la languette 11b, et deux secteurs latéraux 12b, 12c non fixés. Les coutures 13a, 13c sont réalisées le long des bords du secteur proximal 12a et du secteur distal 12d. Les bords externes des secteurs latéraux 12b, 12c sont donc laissés libres. A noter que la couture 13b fixant le bord intérieur de la partie annulaire 11a au manchon 14 sert simplement à éviter la formation de bâillements et peut donc être omise totalement, ou partiellement en conservant les parties situées sur les secteurs proximal 12a et distal 12d.

Au lieu d'être cousus, les secteurs proximal 12a et distal 12d de la plaquette 11 peuvent être fixés sur le manchon 14 par une couche de colle, les secteurs latéraux 12b, 12c n'étant pas collés sur le manchon 14.

Les figures 5A, 5B représentent l'orthèse 10 et la plaquette 11 assemblée sur l'orthèse. La plaquette 11 comprend une pièce de tissu tissé élastique 11d fixée sur une face de la couche en gel polymère de la plaquette 11, sans refermer l'ouverture 12 de la partie annulaire 11a. L'assemblage de la plaquette 11 et de la pièce de tissu 11d peut être effectué par exemple par collage. L'ensemble de la plaquette 11 comprenant la pièce de tissu 11d et la couche en gel polymère peut être ensuite cousu par les coutures 13a, 13b, 13c à la pièce 17 du manchon 14. Les coutures 13a et 13c peuvent être supprimées en collant la pièce de tissu 11d (collée à la plaquette) à la pièce 17, par une couche de colle répandue sur les secteurs annulaires proximal 12a et distal 12d (délimités sur la figure 6 par les coutures 13a et 13c). La pièce de tissu 11d présente une épaisseur inférieure à 0,5 mm, par exemple égale à 0.4 mm et peut être réalisée par exemple dans le même tissu que la pièce postérieure 16.

La dureté et l'épaisseur de la plaquette 11 peuvent être choisies pour permettre de coudre l'ensemble de la plaquette 11 (avec la pièce de tissu 11d). Par ailleurs, l'adhérence ou le tack de la couche en gel polymère de la plaquette 11 peut être choisi afin d'éviter que cette dernière glisse sur la peau, compte tenu des forces de contention exercées par le manchon 14 sur le genou.

Selon un mode de réalisation illustré sur la figure 6, les secteurs annulaires 12a à 12d, délimités par les coutures 13a et 13c s'étendent chacun sur environ un quart (à 10% près) de la circonférence de la partie annulaire 11a, et la plaquette présente une raideur suffisamment faible pour autoriser le port sans gêne de l'orthèse pendant de longues périodes en position assise.

Selon un mode de réalisation, la couche en gel polymère de la plaquette 11 est formée dans un gel de silicone obtenu par polymérisation au moins partielle d'un mélange d'huiles de silicone telles que des huiles de polydiméthylsiloxane. Un tel mélange permet d'obtenir une variété de gels de silicone présentant des propriétés différentes notamment de dureté, et d'adhérence, en fonction des proportions respectives des huiles de silicone constituant le mélange, qui définissent le degré de polymérisation du mélange. Ainsi, en ajustant ces proportions, il est possible d'obtenir un gel viscoélastique plus ou moins dur et plus ou moins adhésif. L'ajustement de la dureté de la plaquette 11 peut être effectué en tenant compte d'exigences d'élasticité et de résistance à l'usure sachant que la plaquette sera mécaniquement très sollicitée, et de dureté, en particulier dans le cas où la plaquette est cousue. En effet, si le gel viscoélastique est trop mou, il aura tendance à encrasser les aiguilles formant les coutures. A noter que l'élasticité de la plaquette 11 dépend aussi de sa forme et de ses dimensions, et en particulier de son épaisseur. Ainsi, compte tenu de sa raideur, la couche en gel polymère de la plaquette 11 peut présenter une épaisseur comprise entre 0,25 et 0,5 mm.

L'orthèse peut être mise en place sur une jambe en l'enfilant par le pied et en tirant sur le bord supérieur du manchon 14, à savoir sur la pièce 15a, jusqu'à ce que la plaquette 11 soit placée sur la rotule (figure 3). La raideur naturelle du manchon fixe l'étirement du manchon, qui est très inférieur à la limite élastique des tissus formant le manchon. Il s'avère que l'allongement de la partie du manchon recouvrant la cuisse, lors d'une flexion du genou à 90°, reste inférieur à 20% dans la zone du manchon où la tension est maximum, et reste inférieur à 10%, à 4 cm de cette zone vers la pièce 15a. Cet allongement est très inférieur à l'allongement maximum des tissus formant le manchon. Dans ces conditions, le pliage alternatif du genou pendant la marche ou la course ne sollicite pas beaucoup le maintien du manchon 14 sur la cuisse, assuré par la bande 15a. Il en résulte que les pièces 15a, 15b sont suffisantes pour empêcher l'orthèse de glisser.

Il peut être constaté que les sollicitations élastiques des tissus du manchon 14 sont maximales juste au dessus de la rotule et diminuent vers le haut de la cuisse. Le manchon 14 peut donc être prévu avec une longueur suffisante entre l'emplacement de la plaquette 11 et son bord proximal, de manière à placer la pièce 15a dans une zone de la cuisse où les sollicitations élastiques du manchon 14 sont relativement faibles (zone de faible étirement cutané). Selon un mode de réalisation, la pièce 15a peut présenter une largeur plus grande que celle de la pièce 15b, typiquement une largeur double de celle de la pièce 15b.

Selon un mode de réalisation, le manchon 14 peut être réalisé de manière à couvrir la cuisse sur une longueur de 18 à 28 cm (à 10% près) à partir de l'axe de la rotule.

Les pièces 17, 18a, 18b, 16, 15a et 15b peuvent être réalisées dans des tissus tissés élastiques suivant deux directions perpendiculaires, par exemple suivant la chaine et suivant la trame du tissu, la chaine de ces pièces étant disposée dans l'axe de la cuisse ou de la jambe et la trame suivant une direction perpendiculaire. Ainsi, le tissu formant la pièce 17 du manchon 14 peut présenter une épaisseur comprise entre 0,4 et 0,5 mm, par exemple 0,42 mm, un allongement maximal (suivant la chaine et la trame du tissu) compris entre 80 et 90%, par exemple égal à 85%, et un module d'élasticité (module de Young) à 40% (d'allongement) compris entre 1,75 et 2 N, par exemple égal à 1.8 N pour la trame et 1,95 N pour la chaine. Le tissu formant les pièces latérales 18a, 18b peut présenter une épaisseur comprise entre 0,3 et 0,4 mm, par exemple égale à 0,38 mm, un allongement maximal (suivant la chaine et la trame du tissu) compris entre 90 et 100%, par exemple égal à 92% pour la chaine et 99% pour la trame, et un module d'élasticité à 40% compris entre 1,7 N et 3 N, par exemple égal à 2 N pour la trame et 2,7 N pour la chaine. Le tissu formant la pièce postérieure 16 peut présenter une épaisseur comprise entre 0,2 et 0,3 mm, par exemple égale à 0,27 mm, un allongement maximal (suivant la chaine et la trame du tissu) compris entre 65 et 75%, par exemple égal à 70% pour la chaine et 67% pour la trame, et un module d'élasticité à 40% compris entre 1,7 et 1,8 N, par exemple égal à 1,74 N pour la chaine et 1,73 N pour la trame.

Les tissus formant les pièces proximale 15a et distale 15b peuvent présenter une épaisseur comprise entre 0,5 et 0,7 mm, par exemple égale à 0,67 mm. Les pièces 15a, 15b peuvent être partiellement recouvertes par la couche adhérente 19a, 19b ayant une masse surfacique comprise entre 18 et 22 µg/cm², par exemple égale à 20 µg/cm². Les couches adhérentes 19a, 19b peuvent ainsi présenter une épaisseur comprise entre 0,15 et 0, 25 mm. Il est possible d'atteindre d'aussi faibles valeurs de masse surfacique pour les couches adhérentes 19a, 19b grâce à l'utilisation d'un tissu sans fil mousse pour réaliser les pièces 15a, 15b. Le tissu formant la pièce proximale 15a peut présenter une largeur (suivant l'axe de la jambe) de 70 à 80 mm, par exemple égale à 77 mm. La couche 19a peut présenter une largeur (suivant l'axe de la jambe) comprise entre 50 et 60 mm, par exemple égale à 53 mm. Le tissu formant la pièce proximale 15a peut présenter un module d'élasticité à 40% compris entre 4 et 5 N, par exemple égal à 4,5 N sans enduction adhérente, et compris entre 6 et 7,5 N, par exemple égal à 6,2 N avec l'enduction adhérente 19a. Le tissu formant la pièce distale 15b peut présenter une largeur (suivant l'axe de la jambe) de 40 à 50 mm, par exemple égale à 45 mm. La couche 19b peut présenter une largeur (suivant l'axe de la jambe) comprise entre 20 et 30 mm, par exemple égale à 25 mm. Le tissu formant la pièce distale 15b peut présenter un module d'élasticité à 40% compris entre 2 et 3 N, par exemple égal à 2,6 N sans enduction adhérente, et compris entre 3 et 4 N, par exemple égal à 3,4 N avec l'enduction adhérente 19b. Toutes les caractéristiques numériques ci-avant des pièces 17, 18a, 18b, 16, 15a et 15b sont définies à 5% près.

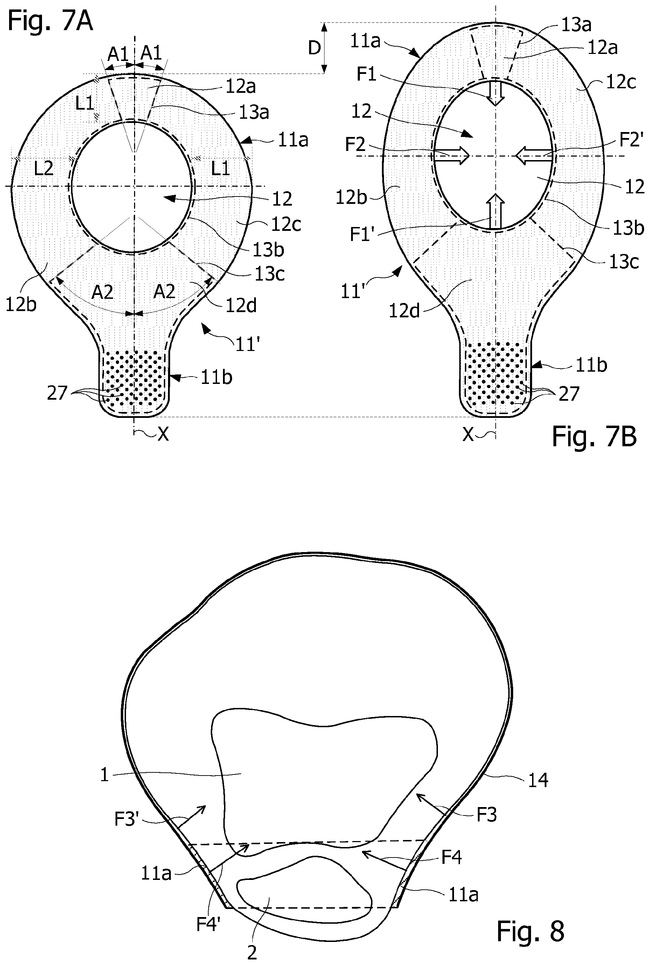

Les figures 7A, 7B représentent une plaquette 11' selon un autre mode de réalisation. Les figures 7A, 7B utilisent les mêmes numéros de référence que sur la figure 6 pour désigner les mêmes éléments. La plaquette 11' diffère de la plaquette 11 par une largeur variable de la partie annulaire 11a, et par des secteurs angulaires 12a à 12d présentant des étendues différentes. Ainsi, la partie annulaire 11a de la plaquette 11' présente, entre ses bords intérieur et extérieur, une largeur L1 dans le secteur angulaire 12a, comprise entre 2,2 et 2,8 cm, par exemple égale à 2,5 cm, et une largeur L2 dans les secteurs angulaires 12b et 12c, comprise entre 2,7 et 3,3 cm, par exemple égale à 3 cm. Les secteurs latéraux 12b, 12c présentent une étendue 4 à 5 fois plus grande que le secteur proximal 12a. Par exemple, le secteur angulaire proximal 12a s'étend sur un angle A1, ayant pour sommet le centre ou un foyer de l'ouverture 12, d'environ 10 à 25°, par exemple compris entre 13 et 20°, de part et d'autre d'un axe longitudinal X de la plaquette 11. Les secteurs latéraux 12b, 12c présentent une étendue comprise entre 1.5 et 2 fois celle du secteur angulaire distal 12d sans la languette 11b. Par exemple, le secteur angulaire distal 12d s'étend sur un angle A2 ayant pour sommet le centre ou un foyer de l'ouverture 12, d'environ 40 à 58° par exemple compris entre 52 et 54°, de part et d'autre de l'axe longitudinal X. La plaquette 11' peut présenter une épaisseur par exemple comprise entre 0,6 et 1 mm. Ces dimensions sont définies à 5% près. Ces dispositions permettent d'allonger la partie étirable de la plaquette, à savoir les secteurs angulaires latéraux 12b, 12c, et ainsi d'augmenter l'élasticité (la capacité d'étirement à force égale d'étirement) de ces secteurs angulaires, sans avoir à diminuer l'épaisseur de la plaquette. En effet, l'assemblage de la plaquette sur le manchon 14 par des coutures peut poser des problèmes de tenue si l'épaisseur de la plaquette est trop faible. Ainsi, les forces exercées par la plaquette 11' sur la rotule sont moindres, malgré une épaisseur plus élevée de la plaquette (par rapport à la plaquette 11), ce qui autorise également de plus longues périodes en position assise sans gêne.

Les figures 7A, 7B représentent la plaquette 11' dans des configurations, respectivement non étirée et étirée, par exemple lorsque l'orthèse est en place sur un membre inférieur, respectivement, genou en extension et genou fléchi. En configuration non étirée, l'ouverture 12 de la plaquette 11' est sensiblement circulaire ou elliptique, adaptée à la forme de la rotule. La languette 11b constitue un point d'ancrage sur la peau, obtenu par la conjonction de la force de contention exercée par le manchon 14 sur la plaquette 11', de la surface de la plaquette et en particulier de la languette 11b, et du coefficient d'adhérence (tack) de la couche en gel polymère de la plaquette. La bande 15a constitue un point d'ancrage du manchon 14 sur la cuisse, et la bande 15b constitue un point d'ancrage du manchon sur la jambe.

Durant l'étirement du manchon 14, lors d'une flexion du genou, une partie des étirements est transmise à la plaquette 11' en raison de son adhérence à la peau, de l'ancrage du manchon 14 par les bandes 15a, 15b, et d'un ancrage supplémentaire de la plaquette 11' résultant en particulier de la liaison par la partie annulaire 11a de la plaquette à la rotule. Il en résulte un étirement D de la plaquette 11' et en particulier de la partie annulaire 11a. L'étirement D entraine une déformation élastique de la plaquette 11', provoquant notamment une déformation de l'ouverture 12 qui s'allonge suivant la direction longitudinale du manchon 14 et se resserre suivant la direction transversale du manchon 14. Il en résulte l'apparition de forces de traction parallèles à la surface de la peau exercées par la plaquette sur la peau et sur le volume du membre qu'elle entoure. Ces forces comprennent des forces longitudinales F1, F1' opposées orientées vers le centre de l'ouverture 12 et des forces transversales F2, F2' opposées, également orientées vers le centre de l'ouverture 12. Les forces F2, F2' assurent le maintien de la rotule et évitent son déjettement latéral.

Les forces F1, F1' participent au déroulement du pas lors de la marche ou la course. Ainsi, durant la marche ou la course, la partie annulaire 11a s'étire pendant une phase active où le pied est posé au sol, tandis que l'inertie du corps participe à la flexion du genou. Pendant une phase passive où le pied ne repose plus sur le sol et le membre inférieur se tend vers l'avant pour prendre un nouvel appui, la partie annulaire 11a reprend sa configuration non étirée et restitue donc à la jambe l'énergie élastique emmagasinée. Même si les forces F2, F2' sont relativement faibles, elles sont suffisantes pour assurer un certain maintien de la rotule et soulager l'articulation en offrant la sensation que l'articulation est tenue (effet proprioceptif de l'orthèse). Quant aux forces F1 et F1', leur présence est perçue par l'intermédiaire du système musculo-tendineux auquel elles s'appliquent.

Il est à noter que la plaquette 11 (figure 6) se comporte de la même manière (précédemment décrite) que la plaquette 11', les forces F1, F1', F2, F2' apparaissant également lorsque la plaquette 11 est étirée.

La figure 8 représente une coupe transversale du genou droit et de l'orthèse l'enveloppant. Il peut être observé que la partie annulaire 11a prend une forme concave sensiblement tronconique, enveloppant les parties latérales droite, gauche, inférieure et supérieure de la rotule 2. La figure 8 montre également par des flèches les forces F3, F3', F4, F4' exercées par l'orthèse sur les bords latéraux de la rotule 2, ces forces étant exercées perpendiculairement à la surface du manchon 14 et de la partie annulaire 11a de la plaquette 11, 11'. Les forces F3, F3' exercées par le manchon 14 sont dirigées vers le centre du genou, et les forces F4, F4' exercées par la partie annulaire 11a de la plaquette 11 sont dirigées sensiblement parallèlement à la surface de l'interface entre la rotule 2 et le fémur 1. L'orthèse permet ainsi d'assurer le maintien latéral de la rotule 2 en particulier lorsque le genou est en extension ou au début d'un mouvement de flexion, c'est-à-dire dans des positions où les tissus qui l'enveloppent sont le moins tendus.

Il est à noter que l'extension longitudinale de la plaquette 11', en particulier de la partie annulaire 11a, notamment en position assise, est en partie facilitée par l'absence de coutures sur le bord externe des secteurs latéraux 12b, 12c de la partie annulaire 11a, relativement étendus, et par la largeur L1 plus faible du secteur angulaire 12a de la partie annulaire 11a. A noter également que le manchon 14, en particulier la pièce antérieure 17, peut présenter une capacité d'étirement longitudinal supérieure aux étirements qui se produisent en position assise, ceci afin d'éviter un glissement des bords proximal et/ou distal du manchon le long de la cuisse ou de la jambe. Si la capacité d'étirement longitudinal de la plaquette 11, 11' et de la pièce 17 sont augmentées, les forces de contention exercées par les pièces 15a, 15b peuvent être diminuées, ce qui contribue à limiter la gêne résultant du port de l'orthèse durant de longues périodes.

Ces dispositions permettent d'obtenir une orthèse de genou de moins de 45 g (à comparer avec celle des orthèses de l'art antérieur qui présentent couramment une masse supérieure à 150 g), tout en assurant un maintien efficace de la rotule compatible avec un usage prolongé durant toute une journée, et avec des activités courantes en mobilité (en dehors d'activités sportives sollicitant le genou) ou statiques, le genou pouvant rester plié jusqu'à 110 degrés durant de longues périodes.

Selon un mode de réalisation, la languette 11b comporte des picots 27 pour augmenter son adhérence à la peau (figures 7A, 7B). Ces picots peuvent être formés par exemple lors de la fabrication par moulage de la plaquette 11, 11'.

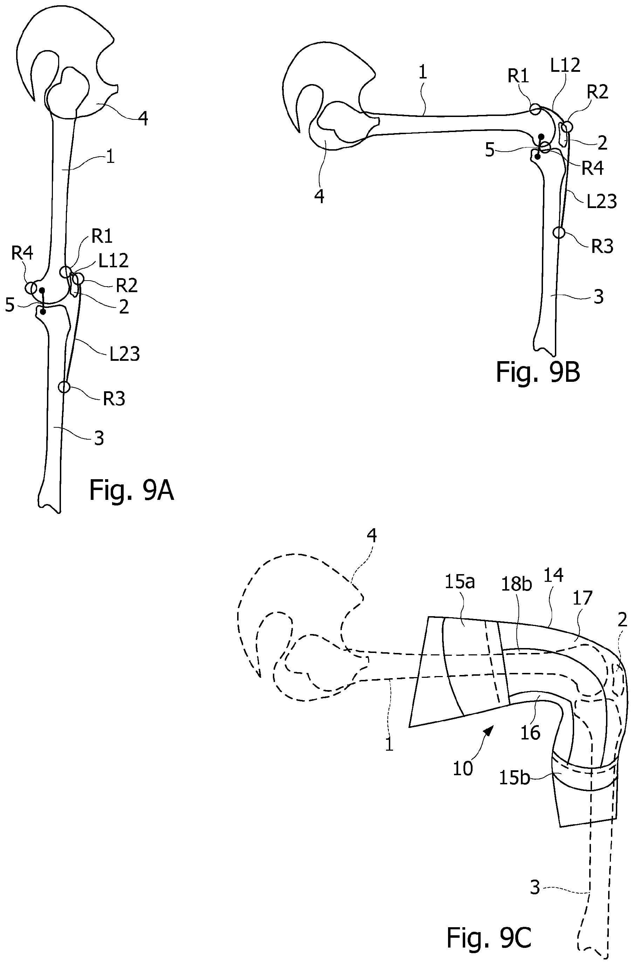

Les figures 9A, 9B représentent l'ossature d'un membre inférieur et du bassin en coupe sagittale (os iliaque 4, fémur 1, tibia 3 et rotule 2), dans des configurations genou en extension et genou fléchi à environ 90°. La figure 9C représente la configuration genou fléchi, superposée à la partie de la jambe équipée de l'orthèse 10. Les figures 9A, 9B symbolisent par des cercles des points repères antérieurs R1, R2, R3 et postérieur R4 sur les os et par des lignes L12, L23, des tissus (muscles, tendons) liant les points de repère R1, R2 et R2, R3. Les points R1 et R4 sont situés respectivement sur les bords antérieur (condyle) et postérieur du fémur 4. Le point R2 est situé sur la rotule 2. Le point R3 est situé en une position distale sur le tibia 3. Les figures 9A, 9B représentent également les ligaments latéraux internes et externes 5 qui sont tendus lorsque le genou est en extension. Pendant une phase de flexion du genou, l'orthèse exerce une force F contribuant à maintenir la rotule 2 et à stabiliser latéralement le genou.

Les figures 9A, 9B montrent que les liaisons antérieures L12 entre les points R1 et R2 et L23 entre les points R2 et R3 subissent des étirements entre les configurations tendue et fléchie du genou. Il en résulte qu'en configuration fléchie (notamment en position assise), la pièce 17 du manchon 14 reste étirée, générant une tension permanente entre la plaquette 11 et la pièce proximale 15a, ainsi qu'entre la plaquette 11 et la pièce distale 15b. Il s'avère que cette tension permanente induit des forces de cisaillement à l'interface entre les manchons 15a, 15b et la peau.

Les caractéristiques d'élasticité et d'adhérence des manchons 15a, 15b, et en particulier le manchon 15a, sont liées à l'élasticité de la partie du manchon 14 la plus sollicitée lors de la flexion de genou, à savoir la pièce 17. Si les manchons 15a, 15b sont insuffisamment adhérents à la peau, la force d'extension appliquée à la pièce 17 lors de la flexion du genou, et transmise aux manchons 15a, provoque un glissement de ces derniers, ce qui n'est pas satisfaisant car le manchon 14 va former des plis et donc ne plus jouer son rôle de maintien de la rotule. A l'inverse, si les manchons 15a, 15b sont très adhérents à la peau, et si la pièce 17 présente une raideur trop élevée, la force d'extension appliquée à la pièce 17, et transmise aux manchons 15a, 15b sous la forme de forces de traction, conduit ces derniers à solliciter la peau sous-jacente au-delà de sa résistance mécanique de cisaillement. Par conséquent, la raideur de la pièce 17 est adaptée pour ne pas solliciter la peau sous les manchons 15a, 15b au-delà de sa résistance mécanique de cisaillement, et la tension et l'adhérence des manchons 15a, 15b autour de la jambe sont fixées à des valeurs telles que les manchons 15a, 15b ne risquent pas de glisser lors de la flexion du genou, compte tenu de la force de traction exercée sur ces derniers par le manchon 14, et en particulier par la pièce 17.

Il apparaîtra clairement à l'homme de l'art que la présente invention est susceptible de diverses variantes de réalisation et diverses applications. En particulier, l'invention n'est pas limitée à une orthèse comprenant les manchons 15a, 15b. En effet, les manchons 15a, 15b peuvent être supprimés partiellement ou en totalité, en remplaçant le manchon 14 par un vêtement de type corsaire ou fuseau serré (pour le genou), couvrant le bassin et au moins un membre inférieur de l'utilisateur devant être maintenu par l'orthèse. Le manchon distal 15b peut être supprimé en ajustant la forme du vêtement, exploitant la forme en fuseau de la jambe au voisinage de la cheville. Il importe simplement que l'orthèse présente des ancrages proximal et distal pour maintenir des parties proximale et distale du manchon 14 en des positions fixes sur le membre inférieur.

## Revendications

1. Orthèse de genou comprenant :
un manchon (14) en tissu tissé élastique, conformé pour exercer des forces de contention sur un membre inférieur de part et d'autre et sur le genou,
une plaquette (11, 11') comprenant une couche viscoélastique, la plaquette étant fixée sur une face interne du manchon de manière à ce que la couche viscoélastique soit en contact direct avec la peau sur le genou, la plaquette comprenant une partie annulaire (11a) conformée pour entourer la rotule du genou, et une languette distale (11b) s'étendant depuis un bord extérieur de la partie annulaire, dans une direction axiale du manchon, la couche viscoélastique présentant une adhérence avec la peau telle que, sous l'effet des forces de contention exercées par le manchon, lorsque le manchon est étiré longitudinalement, la plaquette s'étire et reste étirée en appliquant localement à la partie de membre sous-jacente des forces de maintien en direction du centre du genou, et des forces de rappel dans l'axe du membre, et
des ancrages proximal et distal (19a, 19b), pour maintenir des parties proximale et distale du manchon (14) en des positions fixes sur le membre inférieur,
**caractérisée en ce que** le manchon est formé de pièces de tissu comprenant :
une pièce antérieure (17) sur laquelle est fixée la plaquette (11, 11'),
une pièce postérieure (16) opposée à la pièce antérieure, et présentant un module d'élasticité suivant la direction axiale du manchon, inférieur à celui de la pièce antérieure, et
deux pièces latérales (18a, 18b) fixées chacune à un bord latéral de la pièce antérieure et à un bord latéral de la pièce postérieure, et présentant un module d'élasticité suivant la direction axiale du manchon, supérieur à celui de la pièce antérieure.

2. Orthèse selon la revendication 1, dans laquelle le manchon (14) comprend un manchon proximal (15a) et un manchon distal (15b), en tissu élastique, partiellement recouverts d'une couche (19a, 19b) adhérente à la peau, disposée sur une face interne des manchons proximal et distal, pour venir en contact direct avec la peau et assurer un ancrage sur la peau de bords proximal et distal du manchon, sous l'effet des forces de contention, le manchon proximal étant fixé à un bord proximal de chacune des pièces antérieure (17), postérieure (16) et latérales (18a, 18b), et le manchon distal étant fixé à un bord distal de chacune des pièces antérieure, postérieure et latérales,

3. Orthèse selon la revendication 2, présentant au moins l'une des caractéristiques suivantes :
le manchon proximal (15a) présente une largeur comprise entre 70 et 80 mm, et la couche adhérente (19a) formée sur le manchon proximal, présente une largeur comprise entre 50 et 60 mm,
le manchon distal (15b) présente une largeur comprise entre 40 et 50 mm, et la couche adhérente (19b) formée sur le manchon distal, présente une largeur comprise entre 20 et 30 mm,
les couches adhérentes (19a, 19b) formées respectivement sur les manchons proximal (15a) et distal (15b) présentent une masse surfacique comprise entre 18 et 22 µg/cm²,
les manchons proximal (15a) et distal (15b) sont réalisés dans un tissu élastique ne comportant pas de fil mousse.

4. Orthèse selon l'une des revendications 1 à 3, dans laquelle la plaquette (11, 11') est fixée au manchon (14) par un secteur angulaire proximal (12a) et un secteur angulaire distal (12d) incluant la languette (11b), la plaquette présentant des secteurs angulaires latéraux (12b, 12c) non fixés au manchon.

5. Orthèse selon la revendication 4, dans laquelle la couche viscoélastique de la plaquette (11, 11') présente une épaisseur comprise entre 0,25 et 0,5 mm, et les secteurs angulaires proximal (12a), latéraux (12b, 12c) et distal (12d) s'étendent chacun sur sensiblement un quart de la circonférence de la partie annulaire (11a) de la plaquette (11).

6. Orthèse selon la revendication 4, dans laquelle la couche viscoélastique de la plaquette (11') présente une épaisseur comprise entre 0,35 et 0, 45 mm, et les secteurs angulaires latéraux (12b, 12c) présentent chacun une étendue 4 à 5 fois plus grande que le secteur angulaire proximal (12a) et 1,5 à 2 fois plus grande que le secteur angulaire distal (12d) sans la languette (11b).

7. Orthèse selon la revendication 6, dans laquelle la partie annulaire (11a) de la plaquette (11') présente entre des bords intérieur et extérieur, une largeur (L1) dans le secteur angulaire proximal (12a), comprise entre 2,2 et 2,8 cm, et une largeur (L2) dans les secteurs angulaires latéraux (12b et 12c), comprise entre 2,7 et 3,3 cm.

8. Orthèse selon l'une des revendications 1 à 7, dans laquelle la couche viscoélastique de la plaquette (11, 11') est réalisée en un gel de silicone obtenu par polymérisation au moins partielle d'un mélange d'huiles de polydiméthylsiloxane.

9. Orthèse selon l'une des revendications 1 à 8, dans laquelle la plaquette (11, 11') comprend une couche en tissu élastique (11d) fixée sur la couche viscoélastique.

10. Orthèse selon l'une des revendications 1 à 9, dans laquelle la languette (11b) de la plaquette (11, 11') est conformée de manière à couvrir la tubérosité tibiale, l'orthèse étant adaptée pour être utilisée indifféremment sur un membre inférieur droit ou gauche.

11. Orthèse selon l'une des revendications 1 à 10, présentant au moins l'une des caractéristiques suivantes :
la pièce antérieure (17) du manchon (14) présente une épaisseur comprise entre 0,4 et 0,5 mm,
les pièces latérales (18a, 18b) présentent une épaisseur comprise entre 0,3 et 0,4 mm, et
la pièce postérieure (16) présente une épaisseur comprise entre 0,2 et 0,3 mm.

12. Orthèse selon l'une des revendications 1 à 11, dans laquelle les pièces formant le manchon (14) présentent des modules d'élasticité en présence d'un allongement à 40%, suivant l'axe longitudinal du manchon (14), compris entre 1,75 et 2 N pour la pièce antérieure (17), compris entre 1,7 N et 3 N pour les pièces latérales (18a, 18b), et compris entre 1,7 et 1,8 N pour la pièce postérieure (16).

13. Orthèse selon l'une des revendications 1 à 12, dans laquelle les pièces formant le manchon (14) présentent des modules d'élasticité en présence d'un allongement à 40%, suivant un axe transversal du manchon (14), compris entre 1,75 et 2 N pour la pièce antérieure (17), compris entre 1,7 N et 3 N pour les pièces latérales (18a, 18b), et compris entre 1,7 et 1,8 N pour la pièce postérieure (16).

14. Orthèse selon l'une des revendications 1 à 13, dans laquelle les pièces antérieure (17) et postérieure (16) s'étendent sur environ un tiers de la circonférence du manchon (14) et les pièces latérales (18a, 18b) s'étendent sur environ un sixième de la circonférence du manchon, à 10% près.

## Patentansprüche

1. Eine Knieorthese umfassend:
eine Manschette (14) aus elastischem Gewebe, die so geformt ist, dass sie auf eine untere Extremität von beiden Seiten und auf das Knie Fixationskräfte ausübt, eine Platte (11, 11'), die eine viskoelastische Schicht umfasst, wobei die Platte auf einer Innenfläche der Manschette fixiert ist, so dass die viskoelastische Schicht in direktem Kontakt mit der Haut am Knie ist, wobei die Platte einen ringförmigen Teil (11a) umfasst, der so geformt ist, dass er die Kniescheibe umgibt, und eine distale Zunge (11b), die von einem Außenrand des ringförmigen Teils in axialer Richtung der Manschette verläuft, wobei die viskoelastische Schicht so an der Haut haftet, dass sich die Platte unter der Wirkung der von der Manschette ausgeübten Fixationskräfte, wenn die Manschette längs gedehnt wird, dehnt und gedehnt bleibt, indem lokal auf den darunter liegenden Teil der Extremität Haltekräfte in Richtung der Mitte des Knies und Rückstellkräfte in der Achse der Extremität ausgeübt werden, sowie
proximale und distale Verankerungen (19a, 19b), um proximale und distale Teile der Manschette (14) in festen Positionen an der unteren Extremität zu halten,
**dadurch gekennzeichnet, dass** die Manschette aus Gewebestücken gebildet ist, umfassend:
einen anterioren Teil (17), an dem die Platte (11, 11') befestigt ist,
einen posterioren Teil (16), der sich gegenüber dem anterioren Teil befindet und ein Elastizitätsmodul in axialer Richtung der Manschette aufweist, das sich unterhalb des Elastizitätsmoduls des anterioren Teils befindet und
zwei seitliche Teile (18a, 18b), die jeweils an einem seitlichen Rand des anterioren Teils und an einem seitlichen Rand des posterioren Teils befestigt sind und ein Elastizitätsmodul in axialer Richtung der Manschette aufweisen, das sich oberhalb des Elastizitätsmoduls des posterioren Teils befindet.

2. Orthese nach Anspruch 1, wobei die Manschette (14) eine proximale Manschette (15a) und eine distale Manschette (15b) aus elastischem Gewebe umfasst, die teilweise mit einer Schicht (19a, 19b) bedeckt sind, die an der Haut haftet und auf einer Innenfläche der proximalen und distalen Manschette angeordnet ist, um in direkten Kontakt mit der Haut zu kommen und die proximalen und distalen Ränder der Manschette unter der Wirkung der Fixationskräfte an der Haut zu verankern, wobei die proximale Manschette an einem proximalen Rand von jedem der anterioren (17), posterioren (16) und seitlichen (18a, 18b) Teile und die distale Manschette an einem distalen Rand jedes der anterioren, posterioren und seitlichen Teile befestigt ist.

3. Orthese nach Anspruch 2 mit mindestens einer der folgenden Eigenschaften:
die proximale Manschette (15a) weist eine Breite zwischen 70 und 80 mm auf, und die auf der proximalen Manschette gebildete Haftschicht (19a) weist eine Breite zwischen 50 und 60 mm auf,
die distale Manschette (15b) weist eine Breite zwischen 40 und 50 mm, und die auf der distalen Manschette gebildete Haftschicht (19b) weist eine Breite zwischen 20 und 30 mm auf, wobei die auf der proximalen (15a) bzw. der distalen (15b) Manschette gebildeten Haftschichten (19a, 19b) eine Oberflächenmasse zwischen 18 und 22 µg/cm² aufweisen,
die proximalen (15a) und distalen (15b) Manschetten bestehen aus einem elastischen Gewebe ohne Schaumstofffaden.

4. Orthese nach einem der Ansprüche 1 bis 3, bei der die Platte (11, 11') durch einen proximalen Winkelsektor (12a) und einen distalen Winkelsektor (12d) einschließlich der Zunge (11b) an der Manschette (14) befestigt ist, wobei die Platte seitliche Winkelsektoren (12b, 12c) aufweist, die nicht an der Manschette befestigt sind.

5. Orthese nach Anspruch 4, bei der die viskoelastische Schicht der Platte (11, 11') eine Dicke zwischen 0,25 und 0,5 mm aufweist und sich die proximalen (12a), seitlichen (12b, 12c) und distalen (12d) Winkelsektoren jeweils über im Wesentlichen ein Viertel des Umfangs des ringförmigen Teils (11a) der Platte (11) erstrecken.

6. Orthese nach Anspruch 4, bei der die viskoelastische Schicht der Platte (11') eine Dicke zwischen 0,35 und 0,45 mm aufweist und die seitlichen Winkelsektoren (12b, 12c) jeweils eine 4- bis 5-fach größere Ausdehnung als der proximale Winkelsektor (12a) und eine 1,5- bis 2-fach größere Ausdehnung als der distale Winkelsektor (12d) ohne Zunge (11b) aufweisen.

7. Orthese nach Anspruch 6, bei welcher der ringförmige Teil (11a) der Platte (11') zwischen Innen- und Außenrand im proximalen Winkelsektor (12a) eine Breite (L1) zwischen 2,2 und 2,8 cm und in den seitlichen Winkelsektoren (12b und 12c) eine Breite (L2) zwischen 2,7 und 3,3 cm aufweisen.

8. Orthese nach einem der Ansprüche 1 bis 7, bei der die viskoelastische Schicht der Platte (11, 11') aus einem Silikongel besteht, das zumindest teilweise durch Polymerisation eines Gemisches von Polydimethylsiloxanölen erhalten wird.

9. Orthese nach einem der Ansprüche 1 bis 8, bei der die Platte (11, 11') eine Schicht aus elastischem Gewebe (11d) umfasst, die auf der viskoelastischen Schicht befestigt ist.

10. Orthese nach einem der Ansprüche 1 bis 9, bei der die Zunge (11b) der Platte (11, 11') so geformt ist, dass sie die Tuberositas tibiae bedeckt, wobei die Orthese angepasst ist, um gleichermaßen an einer unteren rechten oder linken Extremität verwendet zu werden.

11. Orthese nach einem der Ansprüche 1 bis 10 mit mindestens einer der folgenden Eigenschaften:
der anteriore Teil (17) der Manschette (14) weist eine Dicke zwischen 0,4 und 0,5 mm auf,
die seitlichen Teile (18a, 18b) weisen eine Dicke zwischen 0,3 und 0,4 mm und der posteriore Teil (16) eine Dicke zwischen 0,2 und 0,3 mm auf.

12. Orthese nach einem der Ansprüche 1 bis 11, bei der die Teile, welche die Manschette (14) bilden, bei einer Dehnung von 40 % entlang der Längsachse der Manschette (14) Elastizitätsmodule zwischen 1,75 und 2 N für den anterioren Teil (17), zwischen 1,7 N und 3 N für die seitlichen Teile (18a, 18b) und zwischen 1,7 und 1,8 N für den posterioren Teil (16) aufweisen.

13. Orthese nach einem der Ansprüche 1 bis 12, bei der die Teile, welche die Manschette (14) bilden, bei einer Dehnung von 40 % entlang einer Querachse der Manschette (14) Elastizitätsmodule zwischen 1,75 und 2 N für den anterioren Teil (17), zwischen 1,7 N und 3 N für die seitlichen Teile (18a, 18b) und zwischen 1,7 und 1,8 N für den posterioren Teil (16) aufweisen.

14. Orthese nach einem der Ansprüche 1 bis 13, bei der sich der anteriore (17) und der posteriore (16) Teil über etwa ein Drittel des Umfangs der Manschette (14) und die seitlichen Teile (18a, 18b) über etwa ein Sechstel des Umfangs der Manschette bis auf 10 % genau erstrecken.

## Claims

1. A knee orthosis comprising:
a sleeve (14) of elastic woven fabric, shaped to exert compressive forces on a lower limb on either side and on the knee,
a pad (11, 11') comprising a viscoelastic layer, the pad being attached to an inner face of the sleeve so that the viscoelastic layer is in direct contact with the skin of the knee, the pad comprising an annular part (11a) shaped to surround the kneecap of the knee, and a distal tab (11b) extending from an outer edge of the annular part, in an axial direction of the sleeve, the viscoelastic layer having an adhesion with the skin such that, under the effect of the compressive forces exerted by the sleeve, when the sleeve is stretched longitudinally, the pad stretches and remains stretched by locally applying to the underlying limb portion support forces towards the center of the knee, and restoring forces in the axis of the limb, and
proximal and distal anchors (19a, 19b) for maintaining proximal and distal parts of the sleeve (14) at fixed positions on the lower limb,
**characterized in that** the sleeve is formed from panels of fabric comprising:
a front panel (17) to which the pad (11, 11') is attached,
a rear panel (16) opposite the front panel, and having a modulus of elasticity in the axial direction of the sleeve, lower than that of the front panel, and
two side panels (18a, 18b), each attached to a lateral edge of the front panel and to a lateral edge of the rear panel, and having a modulus of elasticity in the axial direction of the sleeve, greater than that of the front panel.

2. The orthosis according to claim 1, wherein the sleeve (14) comprises a proximal sleeve (15a) and a distal sleeve (15b), made of elastic fabric, partially covered with a layer (19a, 19b) adhering to the skin, disposed on an inner face of the proximal and distal sleeves, to come into direct contact with the skin and provide an anchorage to the skin of proximal and distal edges of the sleeve, under the effect of the compressive forces, the proximal sleeve being attached to a proximal edge of each of the front (17), rear (16) and lateral (18a, 18b) panels, and the distal sleeve being attached to a distal edge of each of the front, rear and side panels.

3. The orthosis according to claim 2, having at least one of the following features:
the proximal sleeve (15a) has a width between 70 and 80 mm, and the adhering layer (19a) formed on the proximal sleeve has a width between 50 and 60 mm,
the distal sleeve (15b) has a width between 40 and 50 mm, and the adhering layer (19b) formed on the distal sleeve has a width between 20 and 30 mm,
the adhering layers (19a, 19b) formed respectively on the proximal (15a) and distal (15b) sleeves have a surface weight between 18 and 22 µg/cm2,
the proximal (15a) and distal (15b) sleeves are made of an elastic fabric without woolly thread.

4. The orthosis according to one of claims 1 to 3, wherein the pad (11, 11') is attached to the sleeve (14) by a proximal angular sector (12a) and a distal angular sector (12d) including the tab (11b), the pad having lateral angular sectors (12b, 12c) not attached to the sleeve.

5. The orthosis according to claim 4, wherein the viscoelastic layer of the pad (11, 11') has a thickness between 0.25 and 0.5 mm, and the proximal (12a), lateral (12b, 12c), and distal (12d) angular sectors each extend over substantially a quarter of the circumference of the annular part (11a) of the pad (11).

6. The orthosis according to claim 4, wherein the viscoelastic layer of the pad (11') has a thickness between 0.35 and 0.45 mm, and the lateral angular sectors (12b, 12c) each have an extent 4 to 5 times larger than the proximal angular sector (12a) and 1.5 to 2 times larger than the distal angular sector (12d) without the tab (11b).

7. The orthosis according to claim 6, wherein the annular part (11a) of the pad (11') has, between inner and outer edges, a width (L1) in the proximal angular sector (12a), between 2.2 and 2.8 cm, and a width (L2) in the lateral angular sectors (12b and 12c), between 2.7 and 3.3 cm.

8. The orthosis according to one of claims 1 to 7, wherein the viscoelastic layer of the pad (11, 11') is made of a silicone gel obtained by at least partial polymerization of a mixture of polydimethylsiloxane oils.

9. The orthosis according to one of claims 1 to 8, wherein the pad (11, 11') comprises an elastic fabric layer (11d) attached to the viscoelastic layer.

10. The orthosis according to one of claims 1 to 9, wherein the tab (11b) of the pad (11, 11') is shaped to cover the tibial tuberosity, the orthosis being adapted to be used indifferently on a right or left lower limb.

11. The orthosis according to one of claims 1 to 10, having at least one of the following features:
the front panel (17) of the sleeve (14) has a thickness between 0.4 and 0.5 mm,
the side panels (18a, 18b) have a thickness between 0.3 and 0.4 mm, and
the rear panel (16) has a thickness between 0.2 and 0.3 mm.

12. The orthosis according to one of claims 1 to 11, wherein the panels forming the sleeve (14) have an elastic modulus under a 40% elongation, along the longitudinal axis of the sleeve (14), between 1.75 and 2 N for the front panel (17), between 1.7 N and 3 N for the side panels (18a, 18b), and between 1.7 and 1.8 N for the rear panel (16).

13. The orthosis according to one of claims 1 to 12, wherein the panels forming the sleeve (14) have an elastic modulus under an elongation at 40%, along a transverse axis of the sleeve (14), between 1.75 and 2 N for the front panel (17), between 1.7 N and 3 N for the side panels (18a, 18b), and between 1.7 and 1.8 N for the rear panel (16).

14. The orthosis according to one of claims 1 to 13, wherein the front (17) and rear (16) panels extend over approximately one third of the circumference of the sleeve (14) and the side panels (18a, 18b) extend over about one sixth of the circumference of the sleeve, to within 10%.
